# EUROPÄISCHE PATENTSCHRIFT

(11) **EP 0 600 965 B1**
(45) Veröffentlichungstag und Bekanntmachung des Hinweises auf die Patenterteilung: **18.02.1998**
(21) Anmeldenummer: 92917597.4
(22) Anmeldetag: 22.08.1992
(51) Int. Cl.: C12Q 1/68, C07H 21/00

(54) **PRIMER FÜR MATRIZENABHÄNGIGE ENZYMATISCHE NUKLEINSÄURESYNTHESEN**
PRIMERS FOR THE MATRIX-DEPENDANT, ENZYMATIC SYNTHESIS OF NUCLEIC ACIDS
AMORCES POUR LA SYNTHESE ENZYMATIQUE MATRICE-DEPENDANTE D'ACIDES NUCLEIQUES

(30) Priorität: 28.08.1991 DE 4128480
(43) Veröffentlichungstag der Anmeldung: 15.06.1994
(73) Patentinhaber: BOEHRINGER MANNHEIM GMBH, 68298 Mannheim (DE)
(72) Erfinder: SELIGER, Hartmut, D-7915 Elchingen (DE); MARKIEWICZ, Wojciech, T., 61-674 Poznan (PL); GRÖGER, Gabriele, D-7915 Elchingen (DE); RÖSCH, Rudi, D-7900 Ulm (DE); KLOTZ, Margit, D-7933 Schelklingen (DE)
(74) Vertreter: Knauer, Martin, Dr.
(86) Internationale Anmeldenummer: EP9201932
(87) Internationale Veröffentlichungsnummer: WO9305060

(56) Entgegenhaltungen:
- EP-A- 0 423 839
- WO-A-83/02277
- WO-A-86/07361
- WO-A-86/07363
- NUCLEIC ACIDS RESEARCH. Bd. 15, Nr. 7, 10. April 1987, ARLINGTON, VIRGINIA US Seiten 2891 - 2909 J.N.KREMSKY ET AL. 'Immobilization of DNA via Oligonucleotides Containing an Aldehyde or Carboxylic Acid Group at the 5' Terminus.'

## Beschreibung

Techniken der Hybridisierung von Nukleinsäurefragmenten an DNA oder RNA sowie deren matrizenabhängiger enzymatischer Verlängerung, gekoppelt mit deren nicht-radioaktiver Markierung, z. B. durch Fluoreszenzfarbstoffe oder Affinitätsgruppen, sind die Grundlage für viele Anwendungen von Oligonukleotiden im biomedizinischen Bereich. Hierzu gehört z. B. die Sequenzierung von Nukleinsäuren mittels markierter Primer nach dem Sanger'schen Didesoxy-Verfahren oder die Anwendung der Polymerase-Kettenreaktion (PCR) im Zusammenhang mit der Nukleinsäurediagnostik. Nicht-radioaktive Markierungsmethoden sind für solche Zwecke grundsätzlich vorzuziehen, da auf diesem Wege die Probleme der Handhabung von und Kontamination mit Radioisotopen sowie die Schwierigkeiten mit deren Lagerung und Entsorgung vermieden werden.

Die Einführung nicht-radioaktiver Markierungsgruppen kann im Prinzip auf chemischem oder enzymatischem Wege erfolgen. Man muß dabei davon ausgehen, daß die Synthese der Oligonukleotidketten selbst in der Regel auf chemischem Wege durchgeführt wird. Daher ist die nachträgliche enzymatische Markierung, z. B. durch Umsetzung von Oligonukleotiden mit 5-Brom-dUTP (Nucleosides & Nucleotides 8, 805-813 (1989)), Biotin-dUTP (Nucleic Acids Res. 16, 4077-4095 (1988)) oder Digoxigenin-dUTP (Biol. Chem. Hoppe Seyler 371, 917-927 (1000) Katalysiert durch Desoxynukleotidyl-terminaltransferase, von vornherein aufwendig durch die Erfordernis zusätzlicher Verfahrensschritte. Vor allem ist jedoch festzustellen, daß es sich hierbei in der Regel um eine Verlängerung mit strukturell modifizierten Nukleosid-5'-triphosphaten über das 3'-Ende der oligonukleotidkette hinaus handelt. Dabei wird in der Regel automatisch (matrizenunabhängig) mehr als eine Nukleotideinheit an das 3'-Ende angefügt, so daß die so markierten Oligonukleotide für alle matrizenabhängigen Kettenverlängerungen von vornherein nicht zu gebrauchen sind. In DNA 3, 3, Seiten 269 bis 277 (1984) wird ein Verfahren zum enzymatischen Anhängen einer biotinylierten Desoxyuridinrests an das 3'-Ende eines Oligonucleotids beschrieben.

In der EP-A-0 325 970 ist ein Verfahren zur chemischen oder/und enzymatischen Synthese von Oligonucleotiden beschrieben, die an einem Träger mit definierter Porengröße vorgenommen wird.

Auf chemischem Wege kann man Desoxyoligonukleotide nicht-radioaktiv markieren, indem man entsprechende Gruppen entweder an der Nukleobase, am Zuckerrest oder an der Internukleotidbindung (Phosphat) einführt. Dies geschieht zur Zeit fast ausschließlich während oder nach der Synthese und Aufarbeitung des Oligonukleotids. Man substituiert auf diesem Wege entweder das 5'-OH-Ende der Oligonukleotidkette (Nucleic Acid Res. 14, 6227 6245 (1986), nucleic Acids Res. 14, 7985-7994 (1986)), Nukleobasen am 5'-Ende oder intern (Nucleic Acids Res. 15, 3131-3139, (1987); J. Org. Chem. 55, 5931-5933, (1990)) oder 5'-terminale (Nucleic Acids Res. 18, 4355-4360 (1990)) oder internukleotidische (Nucleosides & Nucleotides 10, 303-306 (1991)) Phosphatgruppen. In EP-A-0 423 839 ist ein in N-4-Position markierten Mononucloocid beschrieben, welches zur Bindung an eine Festphase zur Synthese von Oligonucleotidsonden vorgeschlagen wird. Die meiste Markierungsreaktionen erfolgen durch Umsetzungen nach dem chemischen Kettenaufbau. Reaktionspartner ist in diesem Fall die relativ labile Oligonukleotidkette, so daß eine erhebliche Gefahr von Nebenreaktionen besteht. In einigen Fällen werden Markierungsgruppen in der Weise eingeführt, daß man entsprechende Amidophosphitderivate als Reagenzien verwendet. Solche Reagenzien sind in der Regel weniger stabil und in Lösung nur begrenzt lagerfähig. In neuester Zeit sind auch CPG-Trägermaterialien beschrieben worden, die es ermöglichen, gruppen an das 3'-OH-Ende einer Oligonukleotidkette einzuführen (Belmont, Californien, S. 188). Mit den Gruppen ist jedoch das 3'-OH-Ende gegen jede weitere Kettenverlängerung blockiert, so daß auf diese Weise markierte Oligonukleotide als Primer für matrizenabhängige Polymerase-Reaktionen nicht in Frage kommen. Bei allen Oligonukleotiden, die für matrizenabhängige Polymerase-Reaktionen eingesetzt wurden, hat man bisher ausschließlich 5'-terminal markierte Oligonukleotide angewandt. Man ist dabei davon ausgegangen, daß einerseits interne Markierungsgruppen die Hybridisierung stören können, andererseits matrizenabhängige Polymerasen einen Primer nicht über das 3'-Ende hinaus verlängern, wenn dieser dort einen abiologischen Substituenten enthält.

Überraschenderweise fanden wir nun, daß Desoxyoligonukleotide auch dann von DNA-Polymerasen als Primer akzeptiert und in Polynukleotide eingebaut werden, wenn sie Markierungsgruppen, z. B. Fluoreszenzfarbstoffe, an der 3'-terminalen Base enthalten. Der Einbau erfolgt dabei mit gleicher Effizienz wie bei einem mit 5'-terminaler Markierungsgruppe versehenen Primer. Wir haben aufgrund dieses Befundes ein bevorzugtes Verfahren entwickelt, das es ermöglicht, Oligonukleotidsequenzen in markierter Form zu gewinnen und anzuwenden, ohne daß während oder nach der Oligonukleotidsynthese entsprechend funktionalisierte Amidophosphitderivate eingesetzt werden müssen.

Gegenstand der Erfindung ist daher die Verwendung am 3'-Ende markierter Oligonukleotide als Primer zur polymerasekatalysierten Kettenverlängerung.

Unter Markierungsphasen sind polymere Träger zu verstehen, die durch die Art ihrer Funktionalisierung, wie nachstehend beschrieben, einen oder mehrere Substituenten an den Anfang einer Oligonukleotidkette anbringen, die ihrerseits Markierungsgruppen oder Vorstufen derselben sind. Die Substituenten sind so angebracht, daß nach der Oligonukleotidsynthese und dem Abspalten des Oligonukleotids von der Markierungsphase dessen 3'-OH-Gruppe frei ist für die matrizenabhängige Kettenverlängerung mit Polymerasen. Trägermaterialien sind dabei beliebige unvernetzte oder vernetzte Polymere, sowohl organische als auch anorganische Materialien, vorzugsweise Polystyrolderivate, Kieselgel oder controlled pore glass, aber auch lösliche Derivate des Polyethylenglykols, die durch Vorhandensein geeigneter Ankergruppen mit einem Nukleosid oder Nukleotid (bevorzugt über Spacer) verknüpft werden können, wie sie als Träger bei der chemischen Oligonukleotidsynthese üblich sind.

Als Markierungsphasen im Sinne unserer Erfindung sind zu bezeichnen Trägermaterialien der vorher genannten Art, die über einen nicht-nukleosidischen Spacer mit einem mit Markierungsgruppe versehenen Nukleosid oder Nukleotid oder einem Derivat oder einer Vorstufe desselben verbunden sind. In diesem Zusammenhang sind Mononukleoside bevorzugt.

Als nicht-nukleosidische Spacer sind die in der chemischen Oligonukleotidsynthese üblichen Linker zur Verknüpfung von Nukleosiden und Nukleotiden und Trägermaterialien, wie z. B. "long chain alkylamin" Spacer (Oligonukleotide, Synthesis, a practical approach; M.J. Gait, IRL Press 1984, Oxford), einsetzbar.

Eine Ankergruppe ist im Sinne der Erfindung eine chemische Gruppe des Trägers, an die in wässrigem Milieu oder in organischen Lösungsmitteln andere chemische Verbindungen kovalent gebunden werden können. Bevorzugte Ankergruppen sind Aminogruppen, die Hydroxylgruppe oder die Merkaptogruppe. Besonders bevorzugt ist -NH₂.

Der Begriff Markierungsgruppen beschreibt alle kovalent und ggf. über einen Spacer mit dem an der Markierungsphase verankerten Nukleosid verbundenen Substituenten, die es ermöglichen, dem an dieser Fest- oder Flüssigphase hergestellten Oligonukleotid eine besondere strukturelle Eigenschaft zu verleihen, die der Oligonukleotidkette selbst nicht innewohnt. Bevorzugt sind nicht-radioaktive Markierungsgruppen. Vorzugsweise sind dies fluoreszierende, lumineszente, antigene oder affinitätsvermittelnde Gruppen, sowie komplexbildende Gruppen. Desweiteren sind darunter auch solche Gruppen, z.B. Aminogruppen, zu verstehen, die die Anbringung einer Markierung ermöglichen, erforderlichenfalls in geschützter Form. Im weiteren Sinne sollen aber z.B. auch solche Gruppen, die durch ihr Vermögen, als Substituenten einer Oligonukleotidkette DNA oder RNA [oder Proteine] spezifisch zu binden oder zu spalten, einen möglichen therapeutischen Einsatz von Oligonukleotiden erlauben, als Markierungsgruppen im Sinne der Erfindung bezeichnet werden. Besonders bevorzugte Markierungsgruppen sind Haptene, wie Fluorescein, Digoxigenin und Biotin. Haptene sind immunologisch reaktive chemische Verbindungen mit einem Molekulargewicht von weniger als 500 g/mol, die selbst keine Immunantwort auslösen, jedoch dies nach Kopplung an einen hochmolekularen Träger tun.

Als Oligonukleotide im Sinne unserer Erfindung sind sowohl Sequenzen zu verstehen, die aus biologischen Nukleotideinheiten aufgebaut sind, als auch solche, die ganz oder teilweise aus strukturell gegenüber biologischer DNA oder RNA modifizierten Bausteinen und/oder modifizierten Internukleotidbindungen bestehen.

Unter Anwendung der Markierungsphasen können die 3'-markierten Oligonukleotide im übrigen nach üblichen literaturbekannten Methoden und unter Verwendung literaturbekannter Synthons in automatenunterstützten Verfahren hergestellt werden. Dabei ist der Fall nicht ausgeschlossen, daß gleichfalls analog zu literaturbekannten Methoden zusätzlich zu der über die Markierungsphase während der Synthese noch an beliebigen anderen Positionen der Oligonukleotidkette weitere Markierungsgruppen gleicher oder anderer Art eingeführt werden. Die an die Markierungsphase ansynthetisierten Oligonukleotide können jedoch auch nur die üblichen biologischen Nukleinsäurebausteine sein.

Die Oligonukleotidsynthese erfolgt vorzugsweise vom 3'- zum 5'-Ende der gewünschten Sequenz. Bei Benutzung entsprechender Synthons (siehe Beispiel) kann die Synthese alternativ an beliebigen Trägern auch vom 5'- zum 3'-Ende hin durchgeführt werden, wobei 3'-terminal eines oder mehrere Nukleoside eingesetzt werden, bei denen die Nukleobase im Sinne unserer Erfindung mit einer Markierungsgruppe substituiert ist.

Zur Herstellung der Oligonukleotide wird das Trägermaterial mit der Ankergruppe, welche bevorzugt eine Aminogruppe ist, mit einem bevorzugt an der 3'-Hydroxylgruppe aktivierten und an den anderen reaktiven Gruppen (z. B. 5'-OH und primäre Aminogruppen der Nukleobasen) in üblicher Weise geschützten Mononukleosid umgesetzt. Dieses Mononukleosid weist bevorzugt entweder an einer Aminogruppe der Base oder einem zusätzlich an der Base angebrachten Substituenten einen Spacer (Abstandshalter) mit einer endständigen Markierungsgruppe, bevorzugt einer reaktiven und geschützten Gruppe auf.

Für den Fall, daß dieses Nukleosid an der Base eine Gruppe enthält, die in den übrigen Mononukleosideinheiten des zu synthetisierenden Oligonukleotids nicht vorkommt, ist eine selektive Markierung des fertigen Oligonukleotids an dieser Base möglich.

Von der so hergestellten Markierungsphase, welche das geschützte Mononukleosid bevorzugt über einen Spacer an das Trägermaterial gebunden enthält, werden anschließend die 5'-O-Schutzgruppen (z.B. Dimethoxytrityl) abgespalten. Anschließend wird an dieser Markierungsphase eine aus dem Stand der Technik bekannte Oligonukleotidsynthese, bevorzugt nach der Festphasenmethode über Ankopplung von Nukleotidbausteinen über Phosphoramidite durchgeführt, bis die gewünschte Nukleotidsequenz erreicht ist.

Nach der Synthese werden die markierten Oligonukleotidketten gegebenenfalls von Schutzgruppen befreit und so vom Polymerträger abgespalten, daß die Markierungsgruppen oder deren Vorstufen am 3'-Ende der Oligonukleotidkette (und auch an anderen Positionen, sofern sie dort angebracht wurden) verbleiben.

Sofern die Markierungsphase nur eine Vorstufe der Markierungsgruppe enthält bzw. während der Synthese an der 3'-Position nur die Vorstufe einer Markierungsgruppe eingeführt wurde, ist diese in einem abschließenden Schritt in die eigentliche Markierungsgruppe umzuwandeln.

3'-terminal im Sinne unserer Erfindung markierte Oligonukleotide können als Primer in matrizenabhängige Polymerisationen, katalysiert durch DNA- oder RNA-Polymerasen, eingesetzt werden. Dabei können die in der Literatur für die verschiedenen Varianten von matrizenabhängigen Polymerasereaktionen üblichen Reaktionsbedingungen angewandt werden (z.B. EP-A-0 200 362).

Zum Beispiel konnten Primer, die erfindungsgemäß an der 3'-Position mit Fluorescein markiert waren, für die Sequenzanalyse von einzelsträngiger DNA verwendet werden. Die Durchführung der Sequenzierung kann nach der DidesoxyMethode von F. Sanger et al. (Proc. Natl. Acad. Sci. USA 74, 5463-5467 (1977)) entsprechend der Arbeitsvorschrift der Firma Pharmacia (Pharmacia-LKB Autoprimer^{TR} Synthesis Kit (27-9290-01)) erfolgen. In dieser Weise konnten nach Beispiel 4 von pUCl9-DNA ebenso wie in Octopus-DNA D3 Sequenzen bis ca. 500 Basen abgelesen werden.

In Beispiel 5 ist die Sequenzierung für M13mp18 beschrieben, die gleichfalls zur Ablesung entsprechend langer Sequenzen führt.

Zum Beispiel konnten Abschnitte von 141 und 145 Basen der doppelsträngigen M13mpl8RF-DNA durch Anwendung der in Beispiel 6 beschriebenen, jeweils an der 3'-Position mit Fluorescein markierten, Oligonukleotid-Primer (zusammen mit einem nicht markierten Rück-Primer) nach literaturbekannter Vorschrift amplifiziert werden. Nach Gelelektrophorese wurden die amplifizierten DNA-Fragmente durch Ethidiumbromid-Färbung visualisiert. Das Vorhandensein der Fluoresceinreste wurde fluorometrisch nachgewiesen.

Die Anwendung von Markierungsphasen zur Gewinnung markierter Oligonukleotide im Sinne der Erfindung ist insofern von besonderem Vorteil, als diese Phasen nicht nur als Fest- oder Flüssigphasen im Sinne der Merrifield-Synthese den Aufbau von Oligonukleotiden ermöglichen, sondern gleichzeitig den Charakter von stabilen Markierungsreagenzien haben. Damit entfällt die Notwendigkeit zum Einsatz sonstiger bisher während oder nach der Oligonukleotidsynthese einzusetzender Markierungsreagenzien.

Solche Markierungsreagenzien sind häufig nur begrenzt stabil, insbesondere insofern es sich um Amidoposphitreagenzen handelt. Weiterhin ist von besonderem Vorteil, daß bei Anwendung von Markierungsphasen der eigentliche Aufbau der Oligonukleotidkette standardmäßig in automatenunterstützten Verfahren erfolgen kann, ohne daß hierzu zusätzliche Reagenzien oder Syntheseschritte erforderlich sind. Die wesentlichen, wenn nicht alle, Schritte, die zum Einführen der Markierungsgruppe notwendig sind, werden somit vor den Beginn der Oligonukleotidsynthese verlagert.

Mit einer Charge einer Markierungsphase können somit eine Vielzahl beliebiger unterschiedlicher Sequenzen, die das entsprechende markierte Nukleosid (oder mehrere entsprechende Nukleoside) als 3'-Terminus haben, hergestellt werden. Im Gegensatz hierzu mußte bisher jedes Oligonukleotid aus z.B. einer Serie von Sequenzierungsprimern oder von Amplimeren für die PCR-Diagnostik einzeln für sich allein markiert werden. Die Anwendung von Markierungsphasen und der damit gewonnene 3'-markierte Oligonukleotide ermöglicht also eine Rationalisierung aller jener Verfahren, die auf matrizenabhängigen Polymerasereaktionen mit den 3'-terminal markierten Oligonukleotiden als Primern beruhen, insbesondere den Sequenzierungsreaktionen nach Sanger oder der PCR.

Dies ergibt sich aus der Tatsache, daß für die nicht-radioaktive Markierung eines Satzes von n Sequenzierungsprimern nicht mehr, wie bisher, mindestens n Markierungsreaktionen oder Markierungsschritte benötigt werden, sondern nur noch 4 Markierungsphasen. Bei Ausnutzen von "wobble"-Paarungen (I mit T und C, U mit A und G) reduziert sich die Erfordernis auf 2 Markierungsphasen. Stellt man den Restaufwand an Markierungsreaktionen während der Herstellung der Markierungsphasen in Rechnung, dann reduziert sich also der Arbeitsaufwand für die Markierung von Oligonukleotiden auf ca. 2/n bis 4/n. Das heißt, um ein Beispiel zu geben, daß für die Herstellung eines Satzes von 100 sequenziell unterschiedlichen Primern 96-98 % der Markierungsarbeit eingespart werden kann. Werden z.B. für ein größeres Sequenzierungsvorhaben 1000 Primer benötigt, dann steigt die Einsparung sogar auf 99,6 - 99,8 % usw.

In FIG 1 ist die Synthese eines mit Tosyl modifizierten Desoxycytidin-Trägers, die Freisetzung des modifizierten Oligonukleotids und die Umsetzung mit einer Reportergruppe (hier Fluorescein) gezeigt.

In FIG 2 ist das gleiche Verfahren für Desoxyadenosin und Desoxyguanosin gezeigt.

In FIG 3 ist, ausgehend von einem halogenierten Nukleosid-Derivat, die Synthese eines Desoxyuridin-Trägers gezeigt, der (bereits) den Spacer enthält.

FIG 4a betrifft die Sequenzierung von pUC-DNA mit Primer RP.

FIG 4b betrifft die Sequenzierung von pUC-DNA mit Primer A.

FIG 4c betrifft die Sequenzierung von pUC-DNA mit Primer B.

FIG 5a betrifft die Sequenzierung von M13-DNA mit Primer D.

FIG 5b betrifft die Sequenzierung von M13-DNA mit Primer E.

Die folgenden Beispiele erläutern die Erfindung näher.

### Beispiel 1

### Synthese geschützter, markierter Nukleoside für die Gewinnung der Markierunosphasen

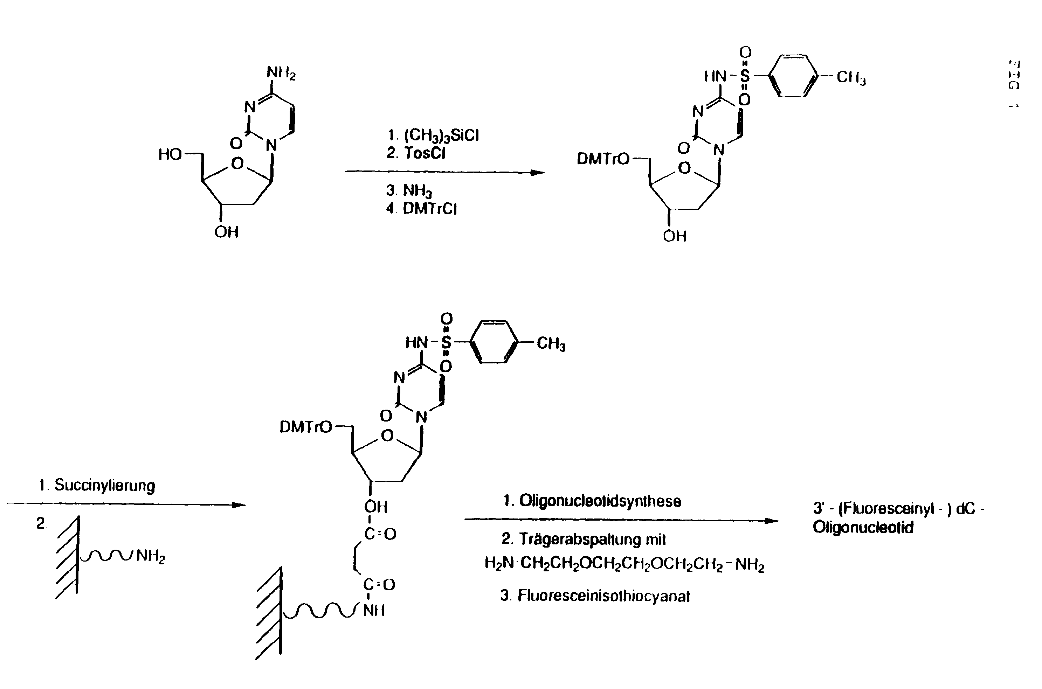

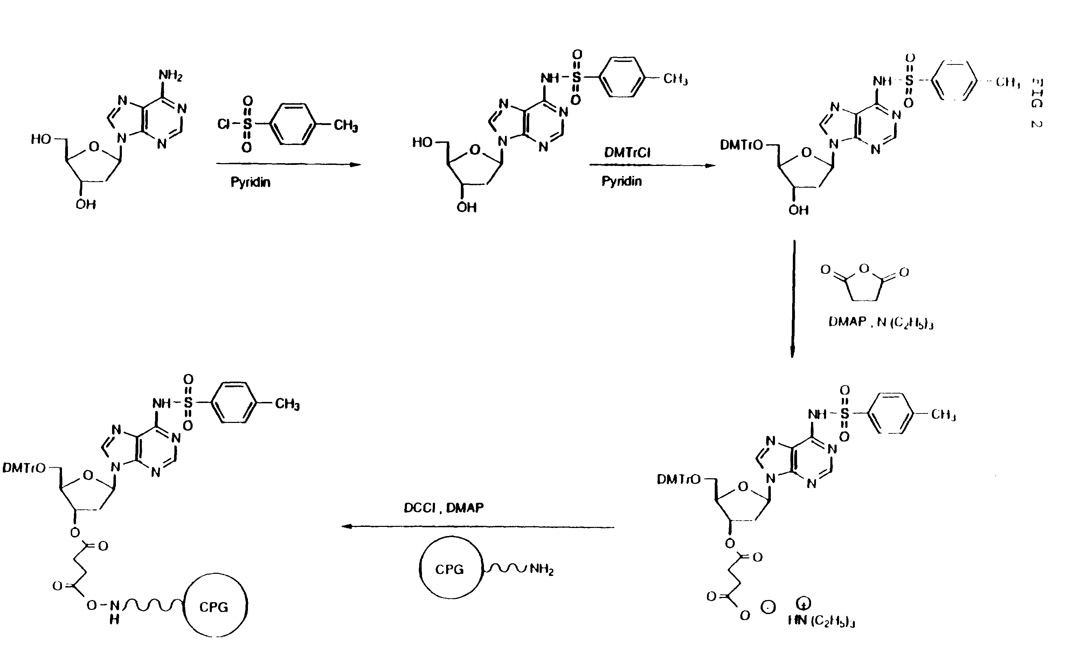

### a) Desoxycytidinträger (FIG 1)

### aa) Synthese von 5'-O-Dimethoxytrityl-4-N-p-toluolsulfonyl-2'-desoxycytidin

2'-Desoxycytidinhydrochlorid (1,32 g, 5 mmol) wird mit Trimethylsilylchlorid (2,5 ml, 20 mmol) in wasserfreiem Pyridin (20 ml) unter Rühren 1 Stunde lang reagieren gelassen. Dann wird die Reaktionsmischung unter Feuchtigkeitsausschluß bis auf ein Volumen von ca. 15 ml eingeengt und p-Toluolsulfonylchlorid (1,9 g, 10mmol) zugegeben. Das gut verschlossene Reaktionsgefäß wird über Nacht bei 60°C im Trockenschrank aufbewahrt. Die dünnschicht-chromatographische Untersuchung des Gemischs zeigt eine vollständige Umsetzung der Edukte. Das Reaktionsvolumen wird durch Zugabe von Dichlormethan (50 ml) vergrößert und die organische Phase gegen eine gesättigte wässrige Natriumbicarbonat-Lösung (60 ml) ausgeschüttelt. Die wässrige Schicht wird zweimal mit Dichlormethan (20 ml) rückextrahiert. Die vereinigten Extrakte werden im Wasserstrahlvakuum konzentriert und mit Pyridin auf ein Volumen von ca. 15 ml aufgefüllt. Konzentrierte wässrige Ammoniaklösung (15 ml) wird zugegeben und der Fortschritt der Desilylierung dünnschichtchromatographisch verfolgt. Die Umsetzung ist nach 4 Stunden quantitativ. Dann wird die Reaktionsmischung unter vermindertem Druck konzentriert und das erhaltene Rohprodukt 4-N-p-Toluolsulfonyl-2'-desoxycytidin wird durch Koevaporation mit wasserfreiem Pyridin (3 x 20 ml) konzentriert und anschließend in absolutem Pyridin (20 ml) wieder gelöst. 4,4'-Dimethoxytritylchlorid (1,70 g, 5 mmol) wird zugegeben. Die Tritylierungsreaktion wird dünnschichtchromatographisch beobachtet und ist innerhalb von 2 Stunden komplett. Die Reaktionsmischung wird zwischen Dichlormethan (50 ml) und 0,5 M wässriger Natriumbicarbonatlösung (50 ml) verteilt. Die wässrige Schicht wird zusätzlich mit Dichlormethan (2 x 50 ml) extrahiert und die organischen Extrakte werden über wasserfreiem Natriumsulfat getrocknet und konzentriert. Reines 5'-O-Dimethoxytrityl-4-N-p-toluolsulfonyl-2'-desoxycytidin wird nach chromatographischer Reinigung an einer Kieselgelsäule mit Dichlormethan/Methanol als Elutionsmittel erhalten. Das Produkt wird in Benzol (20 ml) gelöst, eingefroren und im Ölpumpenvakuum lyophilisiert. Das Reinprodukt wird als weißer Feststoff isoliert. 2,520 g, 73,7 % Ausbeute.

### ab) Synthese des Triethylammoniumsalzes von 5'-O-Dimethoxytrityl-4-N-p-toluolsulfonyl-2'-desoxycytidin -3'-O-monosuccinat

5'-O-Dimethoxytrityl-4-N-p-toluolsulfonyl-2'-desoxycytidin (128 mg, 0,187 mmol), Bernsteinsäureanhydrid (50 mg, 0,5 mmol) und 4-N,N-Dimethylaminopyridin (DMAP, 3 mg, 0,02 mmol) werden mit Triethylamin (0,14 ml, 1 mmol) in wasserfreiem Dichlormethan unter Feuchtigkeitsausschluß gerührt. Die dünnschichtchromatographische Analyse zeigt, daß die Umsetzung innerhalb von 2 Stunden vollständig ist. Die Reaktionsmischung wird zwischen wässrigem Natriumbicarbonat (10 ml) und Dichlormethan (10 ml) verteilt. Die wässrige Schicht wird mit Dichlormethan (2 x 10 ml) rückextrahiert, die kombinierten Extrakte werden über wasserfreiem Natriumsulfat getrocknet und konzentriert.

Der verbleibende Rückstand des Rohproduktes wird in 1,4-Dioxan (3 ml) gelöst, eingefroren und unter Ölpumpenvakuum lyophilisiert. Das gewünschte Produkt entsteht als weißer Feststoff: 165 mg, 99 % Ausbeute.

### ac) Darstellung von 5'-O-Dimethoxytrityl-4-N-p-toluolsulfonyl-2'-desoxycytidin 3'-(2-cyanoethyl)-N,N-diisopropylphoshporamidit

5'-O-Dimethoxytrityl-4-N-p-toluolsulfonyl-2'-desoxycytidin (1,368 g, 2 mmol) wird in wasserfreiem Dichlormethan (10 m) in Gegenwart von Diisopropylethylamin (2 ml, 11,5 mmol) mit dem Magnetrührer gerührt und N,N-Diisopropylamino-(2-cyanoethoxy)-chlorphosphin (0,535 ml, 2,40 mmol) wird zu der beschriebenen Mischung unter Feuchtigkeitsausschluß zugegeben. Die Reaktion ist innerhalb von ca. 15 min. quantitativ, wie durch DC-Analyse gezeigt werden kann. Die Umsetzung wird durch Zugabe von Methanol (0,1 ml) abgebrochen und die Reaktionsmischung mit Dichlormethan (30 ml) versetzt und gegen wässrige Natriumbicarbonat-Lösung (20 ml) ausgeschüttelt. Die wässrige Phase wird mit Dichlormethan (2 x 10 ml) rückextrahiert und die vereinigten Extrakte über wasserfreiem Natriumsulfat getrocknet. An einer Kieselgelsäule mit einer Mischung von n-Hexan-Aceton-Triethylamin mit der Endzusammensetzung von 10:85:5 (durch Vol.) als Elutionsmittel wird das Rohprodukt chromatographisch gereinigt. Das gewünschte Reinprodukt wird durch Lyphilisation aus Benzol (20 ml) als weißes Pulver isoliert.

### ad) Synthese von 5'-O-Dimethoxytrityl-4-N-(2,2'-(ethylendioxyl)-diethylamino)-2'-desoxycytidin

Wasserfreies 2,2'-(Ethylendioxy)-diethylamin (1,2 ml, 12 mmol) werden zu einer Lösung aus 5'-O-Dimethoxytrityl-4-N-p-toluolsulfonyl-2'-desoxycytidin (205 mg, 0,3 mmol) in 7,5 ml Pyridin gegeben. Das Reaktionsgefäß wird dicht verschlossen über Nacht bei 50° C in den Ofen gestellt. Nach Abkühlung auf Raumtemperatur wird das Reaktionsgemisch mit 15 ml Wasser und 7,5 ml Dichlormethan extrahiert. Die organische Phase wird über wasserfreiem Natriumsulfat getrocknet und eingeengt. Die weitere Reinigung erfolgt mittels Kieselgelchromatographie mit einem Gradient aus Dichlormethan/ Methanol. Ausbeute 170 mg (85 % d.Th.) farbloser Schaum.

### ae) Synthese von 5'-O-Dimethoxytrityl-4-N-[8-(fluorescein-4-yl)thioureido-2,2'-(ethylen-dioxy)-diethylamino]-2' -desoxycytidin

5'-O-Dimethoxytrityl-4-N-(2,2(-ethylendioxy)-diethylamino)-2'-desoxycytidin (66 mg, 0,01 mmol) werden in 1 ml DMF gelöst. Dazu gibt man 0,1 ml Natriumboratpuffer (1 M, pH 9,2) und Fluoresceinisothiocyanat (38 mg, 0,01 mmol). Man läßt den Ansatz über Nacht im Dunkeln bei RT stehen. Die Reaktionsmischung wird mit Natriumboratpuffer (10 ml) und Dichlormethan (10 ml) extrahiert. Die Emulsion wird durch Zentrifugation entmischt und die organische Phase abgetrennt. Die Extraktion wird noch zweimal wiederholt. Die vereinigten organischen Phasen werden über Natriumsulfat getrocknet, das Filtrat eingeengt und das Produkt mittels Reverse Column Chromatographie (Merck, RP-2 Kieselgel) mit einer Mischung aus Aceton und gesättigter wässriger Natrium-hydrogencarbonat-Lösung gereinigt. Man erhält 119 mg des Produkts als orangefarbenes Öl.

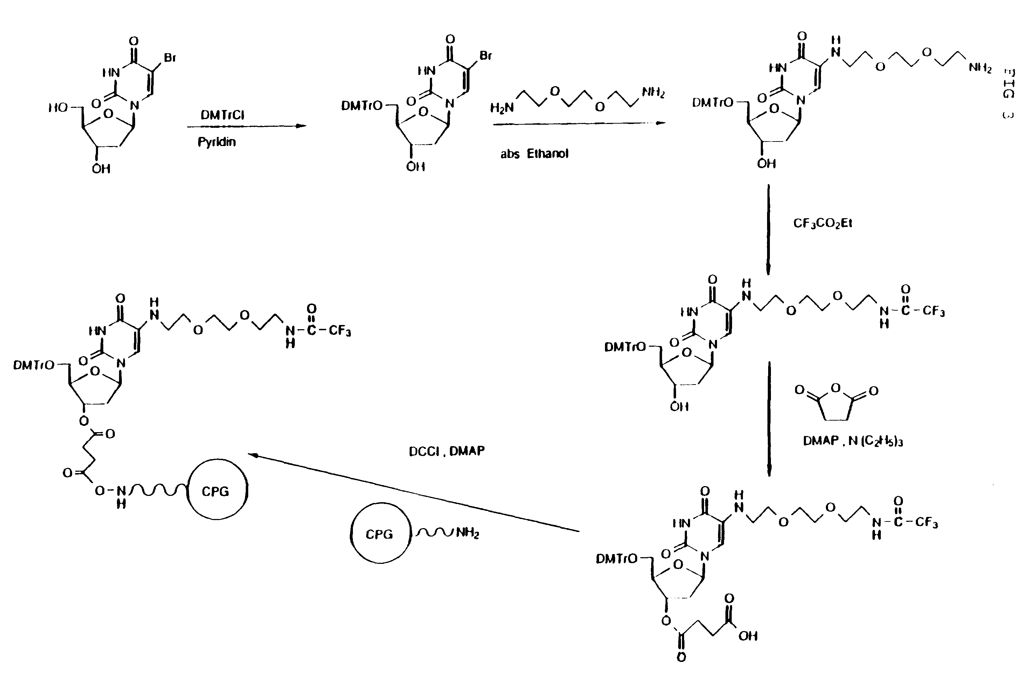

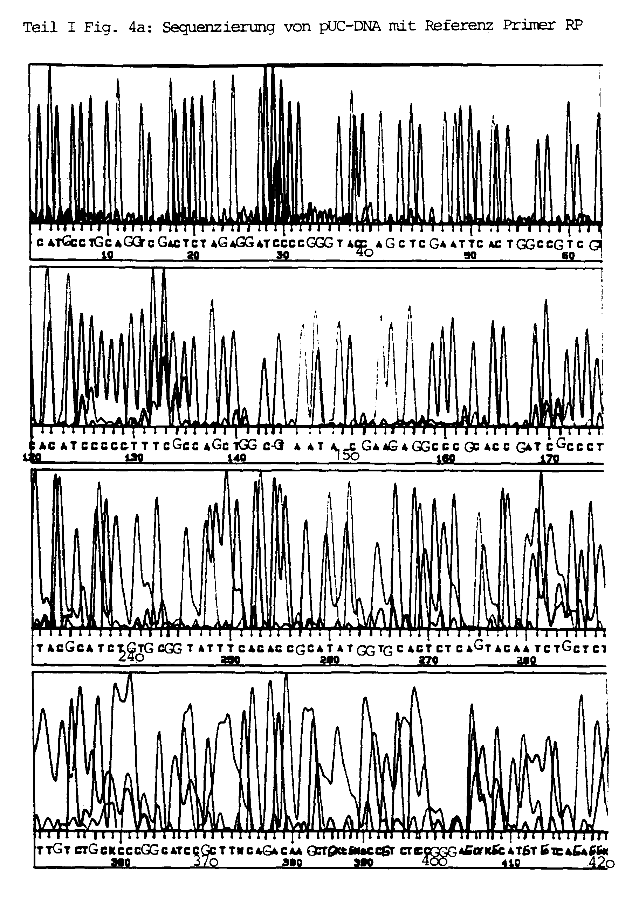

### b) Desoxyadenosinträger (FIG 2)

### ba) Synthese von 6-N-p-Toluolsulfonyl-2'-desoxyadenosin

Desoxyadenosin (1,15 g, 4,0 mmol) wird dreimal mit jeweils 10 ml wasserfreiem Pyridin coevaporiert und anschließend mit Trimethylsilylchlorid (4 ml, 32 mmol) in Pyridin (120 ml) eine Stunde lang bei Raumtemperatur gerührt. Das Gemisch wird dann unter Feuchtigkeitsausschluß auf ein Volumen von ca. 100 ml eingeengt. p-Toluolsulfonylchlorid (1,83 g, 9,5 mmol) und Dimethylaminopyridin (1,0 g, 9,3 mmol) werden zugegeben und unter Feuchtigkeitsausschluß 50 Stunden lang bei 60°C unter Rückfluß gerührt. Zur Umsatzkontrolle wird eine Probe entnommen, mit konzentriertem Ammoniak desilyliert (ca. 2 Stunden), einrotiert und in Dichlormethan aufgenommen. Die dünnschichtchromatographische Untersuchung (Laufmittel Methylenchlorid/ Methanol = 9:1) zeigt, daß ein vollständiger Umsatz nicht erreicht werden kann; die Reaktion wird daher abgebrochen, sobald die Zersetzungsprodukte zunehmen. Das Reaktionsvolumen wird durch Zugabe von Dichlormethan (100 ml) vergrößert, das Gemisch anschließend gegen eine gesättigte Natriumbicarbonatlösung (250 ml) ausgeschüttelt und die wässrige Phase mit Dichlormethan (2 x 50 ml) rückextrahiert. Die organischen Extrakte werden über Natriumsulfat getrocknet und das Volumen anschließend im Wasserstrahlvakuum auf ca. 100 ml eingeengt. Konzentrierte wässrige Ammoniaklösung (100 ml) wird zugegeben und der Verlauf der Desilylierung dünnschichtchromatographisch verfolgt; die Umsetzung ist nach 4 Stunden beendet. Da eine große Anzahl von Zersetzungsprodukten entsteht, wird das Rohprodukt über eine Schwerkraftsäule mit Kieselgel (Merck 7734) und Methylenchlorid/Methanol 10:1 als Elutionsmittel gereinigt.

### bb) Synthese von 5'-O-Dimethoxytrityl-6-N-p-toluolsulfonly-2'-desoxyadenosin

6-N-p-Toluolsulfonyl-2'-desoxyadenosin (4,0 g, 9,8 mmol) wird mit absolutem Pyridin coevaporiert (3 x 15 ml), anschließend in Pyridin (50 ml) aufgenommen und 4,4'-Dimethoxytritylchlorid (3,75 g, 11 mmol) unter Argon zugegeben. Die dünnschichtchromatographische Kontrolle zeigt, daß die Umsetzung nach 2 Stunden vollständig ist. Dichlormethan (50 ml) wird zugegeben und das Gemisch gegen 0,5 M Natriumhydrogencarbonatlösung ausgeschüttelt. Die wässrige Phase wird mit Dichlormethan (2 x 50 ml) reextrahiert, die vereinigten organischen Extrakte über Natriumsulfat getrocknet und einrotiert. Das so erhaltene Rohprodukt wird chromatographisch an einer Kieselgelsäule (Merck H 60) mit Methylenchlorid/Methanol als Elutionsmittel gereinigt.

### bc) Synthese des Triethylammoniumsalzes von 5'-O-DMTr-6-N-p-toluolsulfonyl-2'-desoxyadenosin-3'-O-monosuccinats

5'-O-DMTr-6-N-p-toluolsulfonyl-2'-desoxadenosin (200 mg, 0,28 mmol), Bernsteinsäureanhydrid (70 mg, 0,7 mmol), 4-N,N-Dimethylaminopyridin (DMAP, 4,5 mg, 0,03 mmol) und absolutes Triethylamin (0,2 ml, 1,4 mmol) werden in absolutem Methylenchlorid gelöst und bei Raumtemperatur 2 Stunden gerührt. Die Reaktionsmischung wird dann zwischen wässrigem Natriumbicarbonat (10 ml) und Dichlormethan (10 ml) verteilt, die wässrige Phase mit Dichlormethan (2 x 10 ml) reextrahiert, die vereinigten organischen Phasen über Natriumsulfat getrocknet und einrotiert. Das so erhaltene Produkt wird ohne Aufreinigung weiterverarbeitet.

### bd) Synthese von 5'-O-Dimethoxytrityl-8-(2,2'-(ethylendioxy)-diethylamino)-2'-desoxyadenosin

Wasserfreies 2,2-(Ethylendioxy)-diethylamin (0,5 ml) wird zu einer Lösung aus 5'-O-Dimethoxytrityl-8-bromo-2'-desoxyadenosin (100 mg, 0,15 mmol) in 0,5 ml Pyridin gegeben. Das Reaktionsgefäß wird dicht verschlossen und über Nacht bei 70°C in einen Ofen gestellt. Die weitere Aufarbeitung und Reinigung ist analog zu der Prozedur für 5'-O-Dimethoxytrityl-4-N-2,2'-(ethylendioxy)-diethylamino)-2'-desoxycytidin wie in Beispiel ad).

### be) Synthese von 5'-O-Dimethoxytrityl-8-8-(fluorescein-4-yl)thioureido-2,2'-(ethylendioxy)-diethylamino)-2' -desoxyadenosin

5'-O-Dimethoxytrityl-8-N-(2,2'-(ethylendioxy)-diethylamino)-2'-desoxyadenosin (82 mg, 0,12 mmol) werden in 1 ml DMF gelöst. Dazu gibt man 0,1 ml Natriumborat-puffer (1 M, pH 9,2) und Fluoresceinisothiocyanat (47 mg, 0,12 mmol). Man läßt den Ansatz über Nacht im Dunkeln bei RT stehen. Die Reaktionsmischung wird mit Natriumboratpuffer (10 ml) und Dichlormethan (10 ml) extrahiert. Die Emulsion wird durch Zentrifugation entmischt und die organische Phase abgetrennt. Die Extraktion wird noch zweimal wiederholt. Die vereinigten organischen Phasen werden über Natriumsulfat getrocknet, das Filtrat eingeengt und das Produkt mittels Reverse Column Chromatographie (Merck, RP-2 Kieselgel) mit einer Mischung aus Aceton und gesättigter wässriger Natriumhydrogencarbonat-Lösung gereinigt. Man erhält 97 mg des Produkts als orangefarbenes Öl.

### c) Desoxyguanosinträger

Die Synthese von 5'-O-DMTr-2-N-p-toluolsufonyl-2'-desoxyguanosin wird analog bb) durchgeführt (FIG 2).

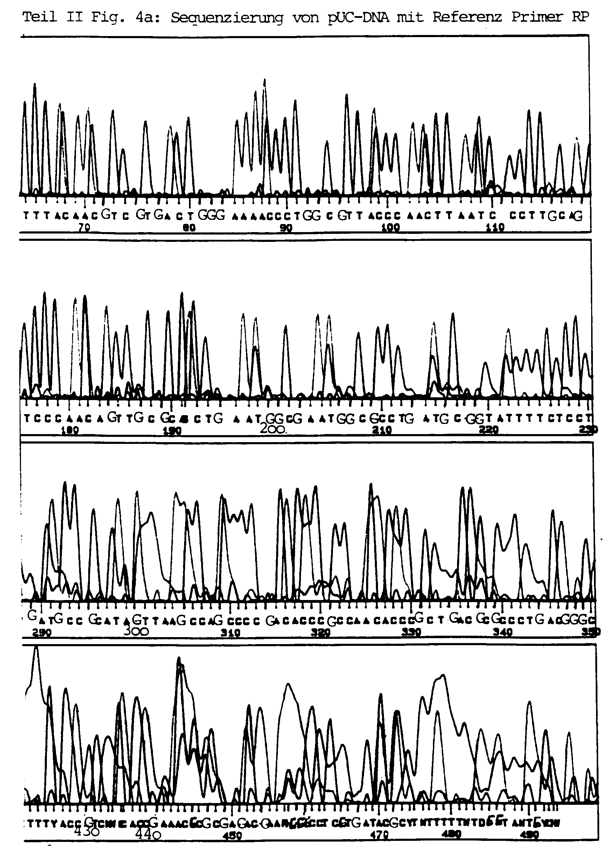

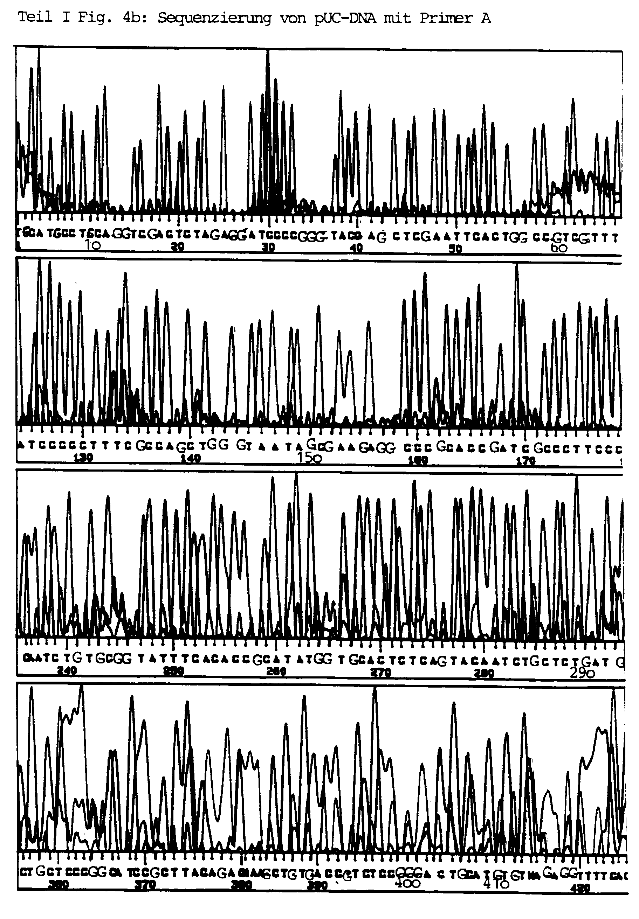

### d) Desoxyuridinträger (FIG 3)

### da) Synthese von 5'-O-Dimethoxytrityl-5-bromo-2'-desoxyuridin

5-Bromo-2'-desoxyuridin (4,0 g, 13 mmol) wird mit absolutem Pyridin (3 x 15 ml) koevaporiert, anschließend in Pyridin (50 ml) aufgenommen und 4,4'-Dimethoxytritylchlorid (4,88 g, 14,3 mmol) unter Argon zugegeben. Die dünnschichtchromatographische Kontrolle zeigt, daß die Umsetzung nach 2 Stunden vollständig ist. Dichlormethan (50 ml) wird zugegeben und das Gemisch gegen 0,5 M Natriumbicarbonatlösung ausgeschüttelt. Die wässrige Phase wird mit Dichlormethan (2 x 50 ml) rückextrahiert, die vereinigten organischen Extrakte über Natriumsulfat getrocknet und einrotiert. Das so erhaltene Rohprodukt wird chromatographisch an einer Kieselgelsäule mit Methylenchlorid/ Methanol als Elutionsmittel gereinigt.

### db) Synthese von 5'-O-Dimethoxytrityl-5(2,2'-(ethylendioxy)-diethylamino)-2'-desoxyuridin

5'-O-Dimethoxytrityl-5-bromo-2'-desoxyuridin (0,7 g, 1,2 mmol) werden in absolutem Ethanol (10 ml) unter Feuchtigkeitsausschluß gelöst. Trockenes 2,2'-(Ethylendioxy)-diethylamin (0,7 ml, 4,8 mmol) wird zugegeben und das Gemisch 24 Stunden unter Rückfluß erhitzt. Anschließend wird die Lösung unter Vakuum bis zur Trockene eingeengt. Der ölige Rückstand wird in Dichlormethan (30 ml) aufgenommen und mit gesättigter Kaliumchloridlösung (2 x 30 ml) zur Entfernung des größten Teil des überschüssigen Diamins extrahiert. Die organische Phase wird über Natriumsulfat getrocknet und einrotiert. Zur weiteren Entfernung des Diamins wird der Rückstand mit wenig Wasser (2 - 3 ml) digeriert. Das Wasser wird abgehoben und verworfen. Das Produkt wird im Exsikkator über Phosphorpentoxid solange im Ölpumpenvakuum getrocknet, bis es eine pulvrige Konsistenz annimmt.

### Beispiel 2

### Allgemeine Vorschrift für die Herstellung von Markierungsphasen für die Oligonukleotidsynthese unter Verwendung von Bernsteinsäureestern der geschützten markierten Nukleoside

Das Triethylammoniumsalz des in geeigneter Weise geschützten Nukleosidsuccinates (0,185 mmol, z.B. Beispiel 1 bc), ein Trägermaterial (1,0 g), N,N'-Dicyclohexylcarbodiimid (200 mg, 1 mmol), 4-N,N-Dimethylaminopyridin (DMAP, 6 mg, 0,05 mmol), Triethylamin (0,07 ml, 0,5 mmol) werden in wasserfreiem Dichlormethan (3 ml) über Nacht bei Raumtemperatur unter Luftausschluß geschüttelt. Dann wird der Träger abfiltriert, mit Dichlormethan (50 ml) gewaschen und in den Kolben für die Maskierungsreaktion mit Essigsäureanhydrid (0,35 ml), Triethylamin (1 ml), DMAP (12 mg) in Dichlormethan (2 ml) überführt und 2 Stunden lang reagieren gelassen. Der Träger wird abfiltriert und nacheinander mit Dichlormethan, Methanol, Dichlormethan und Diethylether (jeweils 25 ml) gewaschen und im Exsikkator im Ölpumpenvakuum bis zur Gewichtskonstanz getrocknet.

Der LCAA-CPG 500 A Träger mit 5'-O-Dimethoxytrityl-4-N-p-toluolsulfonyl-2'-desoxycytidin (bzw. -adenosin) wird entsprechend der oben beschriebenen Anleitung mit einer Beladung von 18 µmol/g (bzw. 26 µmol/g beim Adenosinderivat) hergestellt.

### Derivatisierung von "long chain aminoalkyl" controlled pore glass Träger (LCAA-CPG 500) mit 4-N-p-Toluolsulfonyl-2'-desoxycycidin bzw. mit 6-N-p-Toluolsulfonyl-2'-desoxyadenosin

Das Triethylammoniumsalz des 5'-O-Dimethoxytrityl-p-toluolsulfonyl-2'-desoxynukleosids-3'-O-monosuccinat (0,186 mmol), LCAA-CPG 500A (Firma Pierce, 1.000 g), N,N'-Dicyclohexylcarbodiimid (200 mg, 1 mmol), 4-N,N-Dimethylaminopyridin (DMAP, 6 mg, 0,05 mmol) und Triethylamin (0,07 ml, 0,5 mmol) werden in wasserfreiem Dichlormethan (3 ml) vorsichtig mechanisch über Nacht bei Raumtemperatur unter Feuchtigkeitsausschluß geschüttelt. Der Träger wird abfiltriert, mit Dichlormethan (50 ml) gewaschen und in den Reaktionskolben für die Maskierung unreagierter Hydroxylgruppen überführt, mit Essigsäureanhydrid (0,35 ml), Triethylamin (1 ml), DMAP (12 mg) in trockenem Dichlormethan (2 ml) 2 Stunden lang geschüttelt. Der Träger wird abfiltriert, nacheinander mit Dichlormethan, Methanol, Dichlormethan und Diethylether (jeweils 25 ml) gewaschen und in einem Exsikkator im Ölpumpenvakuum bis zur Gewichtskonstanz getrocknet. Die Beladung des Trägers wird durch spektrophotometrische Messung des Dimethoxytritylkations abgeschätzt, die an einer kleinen Probe des Trägers nach Behandlung mit Säure nach der in der Literatur beschriebenen Methode durchgeführt wird und bei 18 µmol/g für das Cytidinderivat und bei 26 µmol/g für das Adenosinderivat liegt.

Analog wird die Umsetzung an anderen mit Aminogruppen beladenen Trägermaterialien durchgeführt (z.B. CPG 1400).

### Derivatisierung von LCAA-CPG 500 mit 5'-O-Dimethoxytrityl-8-(8-fluorexcein-4-yl)thioureido-2,2'-(ethylendioxy) -diethylamino)-2'-desoxyadenosin bzw. mit 5'-O-Dimethoxytrityl-4-N-(8-fluorescein-4-yl)thioureido-2,2' -(ethylendioxy)-diethylamino-2'-desoxycytidin

5'-O-Dimethoxytrityl-8-(8-fluorescein-4-yl)thioureido-2,2-(ethylendioxy)-diethylamino)-2'-desoxyadenosin (55 mg, 0,05 mmol) werden in Dichlormethan/Pyridin (1:1, 3,5 ml) gelöst und Triethylamin (0,3 ml), 4-N,N-Dimethylaminopyridin (DMAP, 12 mg) und Bernsteinsäureanhydrid (200 mg) zugegeben. Man läßt über Nacht im Dunkeln bei RT reagieren. Die Reaktionsmischung wird anschließend eingeengt und mit Dichlormethan/wässriger Natriumhydrogencarbonatlösung extrahiert. Die organische Phase wird nach Trocknen über Natriumsulfat eingeengt und mit Pyridin coevaporiert. Zu diesem so erhaltenen Rohprodukt gibt man LCAA-CPG 500°A (300 mg, Firma Pierce), DMAP (6 mg), Triethylamin (0.07 ml), Dicyclohexylcarbodiimid (DCCJ, 200 mg) und Dichlormethan (1,5 ml). Die Reaktionsmischung wird über Nacht im Dunkeln bei RT geschüttelt, der Träger abfiltriert und mit Dichlormethan, Methanol und Diethylether gewaschen. Danach wird der derivatisierte Träger in einen Rundkolben überführt und Essigsäureanhydrid (0,5 ml), Pyridin (1 ml), DMAP (12 mg) und Dichlormethan (5 ml) zugegeben. Nach 3h wird der Träger abfiltriert, gewaschen und im Exsikkator im Ölpumpenvakuum getrocknet. Die Beladung des Trägers beträgt für das Adenosinderivat 19,6 µmol/g und für das Cytidinderivat 19,5 µmol/g.

Mit diesen Trägern wurden die in Beispiel 5 beschriebenen Primer D und Primer E synthetisiert.

### Beispiel 3

### Allgemeine Arbeitsvorschrift für die Synthese von markierten Oligonukleotiden unter Verwendung von Markierungsphasen

### a) Synthese von Oligonukleotiden mit p-Toluolsulfonylgruppen und ihre Reaktion mit 2,2'-(Ethylendioxy)-diethylamin

oligonukleotide werden mit dem Gene Assembler Plus der Firma Pharmacia-LKB (Schweden) entsprechend dem Protokoll des Herstellers synthetisiert, wobei geeignete Trägermaterialien (0,2 µmol) und Phosphoramidite verwendet werden. Die Oligonukleotide werden in einem Eppendorf Reaktionsgefäß durch Behandlung mit konzentriertem wässrigen Ammoniak bei Raumtemperatur innerhalb von einer Stunde vom Träger abgespalten. Die gesammelte Lösung wird in einem Speed-vac Evaporator bis zur Trockene konzentriert. Wasserfreies 2,2'-(Ethylendioxy)-diethylamin (100 µl) wird zu den teilweise entschützten Oligonukleotiden zugegeben, das Reaktionsgefäß verschlossen und das Oligonukleotid wird bei Raumtemperatur in einem Ultraschallbad (5 bis 10 min) aufgelöst. Die Reaktionslösung wird über Nacht in den Wärmeschrank bei 80°C gestellt. Das bis auf die p-Toluolsulfonylgruppen vollständig entschützte Produkt wird aus der Amin-Lösung gefällt. Das Reaktionsgefäß wird zentrifugiert und die Lösung vorsichtig abgehoben. Wasserfreies Ethanol (100 µl) wird zugegeben, das Präzipitat mit Hilfe eines Ultraschallbades (5 bis 10 min) gewaschen und abzentrifugiert. Der Überstand wird verworfen und die Waschprozedur einmal wiederholt. Der Niederschlag des Rohprodukts wird im Speed-vac Evaporator getrocknet und in weiteren Experimenten eingesetzt.

### b) Reaktion von Aminoalkyloligonukleotiden mit Fluoresceinisothiocyanat

Das Verfahren zur Einführung von Fluorescein-Resten in Oligonukleotide ist im wesentlichen die gleiche wie vom Protokoll der Fa. Pharmacia-LKB (Pharmacia-LKB Autoprimer^{TM} Synthesis Kit (27-9290-01) angegeben wird. Der Niederschlag des ungereinigten Oligonukleotids wird in 0,15 M Natriumborat-Puffer pH 9,2 (100 µl) gelöst und eine frisch hergestellte Lösung von Fluoresceinisothiocyanat Isomer I (1 mg) in wasserfreiem Dimethylsulfoxid (30 µl) zugegeben. Die Reaktion wird in der Dunkelheit bei Raumtemperatur 4 bis 20 Stunden mit im wesentlichen gleichen Resultaten durchgeführt. Die Reaktionsmischung wird auf eine NAP-10 Säule (Pharmacia-LKB) aufgetragen und mit bidestilliertem Wasser (pH 8 bis 9.5, eingestellt mit Ammoniak) gewaschen. Die die Oligonukleotide enthaltenden Fraktionen (1,3 ml) werden gesammelt und das gewünschte, markierte Oligonukleotid nach Polyacrylamidgelelektrophorese (20 %, 7 M Harnstoff) oder durch HPLC isoliert und mit Hilfe von UV-Vis Spektrometrie charakterisiert. Die Spaltung mit Schlangengiftphosphodiesterase / alkalischer Rinderphosphatase wird nach (Anal. Biochem. 165, 442-447 (1987) ) durchgeführt.

### c) Synthese von Oligonukleotiden unter verwendung von Trägern mit Fluoresceinated Desoxynukleotiden

Die Oligonukleotide werden mit dem Gene Assembler Plus der Firma Pharmacia-LKB (Schweden) entsprechend dem Protokoll des Herstellers mit geeigneten Phosphoramiditen synthetisiert. Als Trägermaterial verwendet man die mit Fluorescein-desoxynukleotiden derivatisierten LCAA-CPG 500 A Träger (10 mg, 0,2 µmol). Die Abspaltung des synthetisierten Oligonukleotids und die weitere Aufarbeitung erfolgen analog dem Standard-protokoll von Pharmacia. Die weitere Reinigung erfolgt durch Polyacrylamidgelelektrophorese (20 %, 7 M Harnstoff) oder durch Reversed Phase HPLC.

### Beispiel 4

### Anwendung 3'-terminal markierter Oligonukleotide in der matrizenabhängigen Kettenverlängerung, katalysiert durch DNA-Polymerase

Sequenzierungsuntersuchungen wurden am Beispiel folgender modifizerter Oligonukleotide durchgeführt: 
Primer A:   5'-CAGGAAACAGCTATAGX     SEQ ID 1
Primer B:   3'-XAGGAAACAGCTATGAC     SEQ ID 2
(beide Primer wurden nach 3a, b) hergestellt)
 X = mit Fluorescein markiertes Cytidin

Als Referenzsubstanz (Primer RP) wurde ein in üblicher Weise in 5'-Position mit einem Aminolinker umgesetzter und mit Fluorescein markierter analoger Primer eingesetzt (Anleitung Fa. Pharmacia). Als zu untersuchende DNA wurden pUC 19 sowie Octopus DNA (D3) eingesetzt.

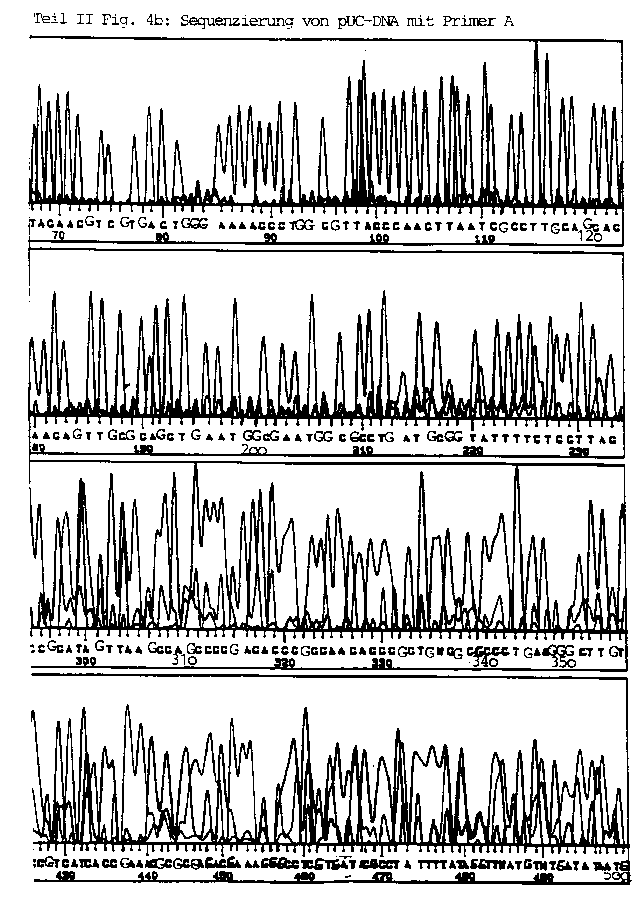

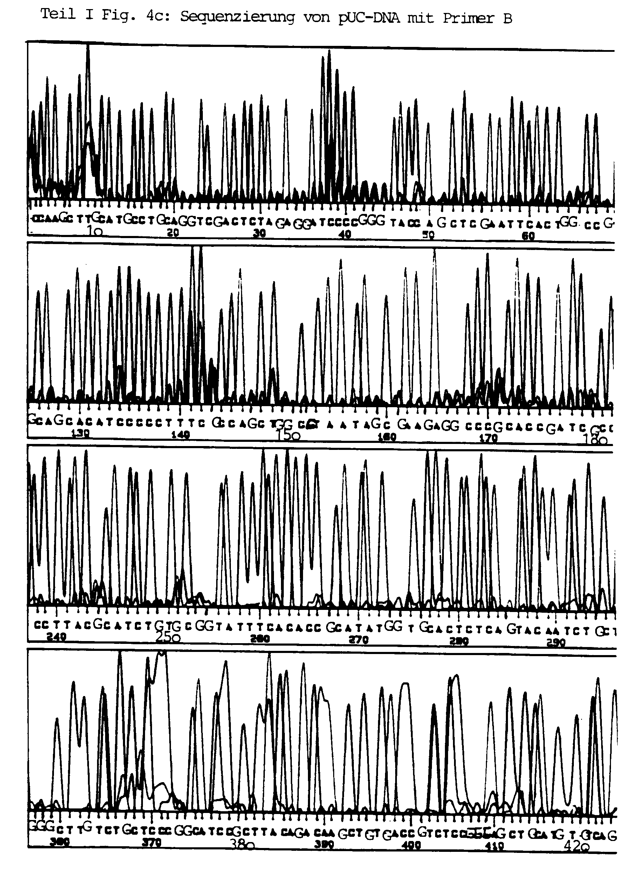

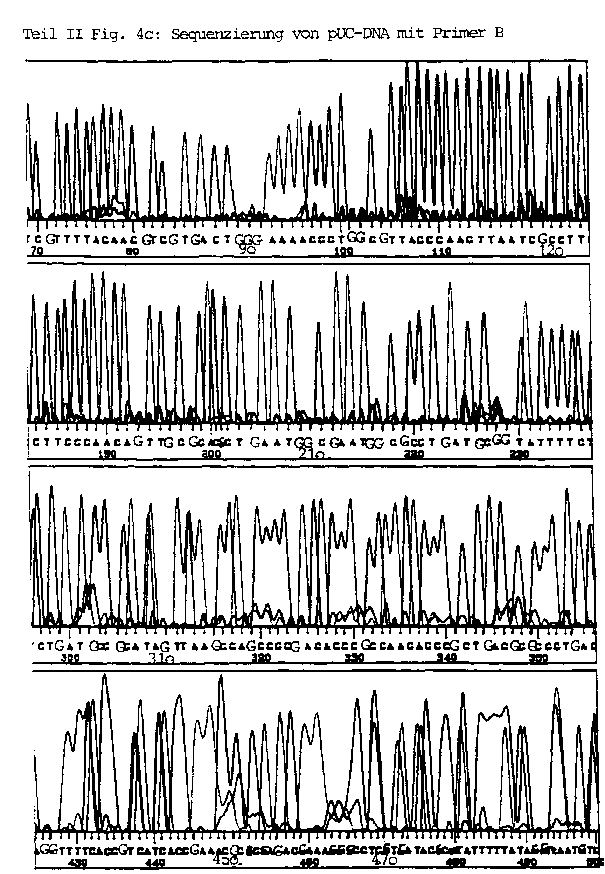

Die Durchführung der Sequenzierung erfolgt für alle Primer nach der Sanger'schen Didesoxymethode nach der Arbeitsvorschrift der Fa. Pharmacia. Die Sequenzierungsergebnisse an pUC 19-DNA sind in den Abbildungen 4a, b und c dargestellt.

Analog wurde mit folgendem Primer sequenziert: Primer C:    5'-TTTCACACAGGAAACAGCTATGY   SEQ ID NO 3 Y = mit Fluorescein markiertes Adenosin.

### Beispiel 5

### Anwendung 3'-terminal markierter Oligonukleotide im M13-Standard-Sequenzierungssystem

Zwei Varianten des Universal-M13-Sequenzierungsprimers wurden für Sequenzierungsuntersuchungen eingesetzt: 
Primer D:        5'-TTGTAAAACGACGGCCY  SEQ ID NO 4
                                      (hergestellt nach
                                       3c)
 
Primer E:      5'-AAAACGACGGCCAGTGX  SEQ ID NO 5

(beide Primer wurden nach 3c) hergestellt)
Y = mit Fluorescein markiertes Adenosin
X = mit Fluorescein markiertes Cytidin

Als zu untersuchende DNA wurde M13mp18 (Fa. Pharmacia) verwendet.

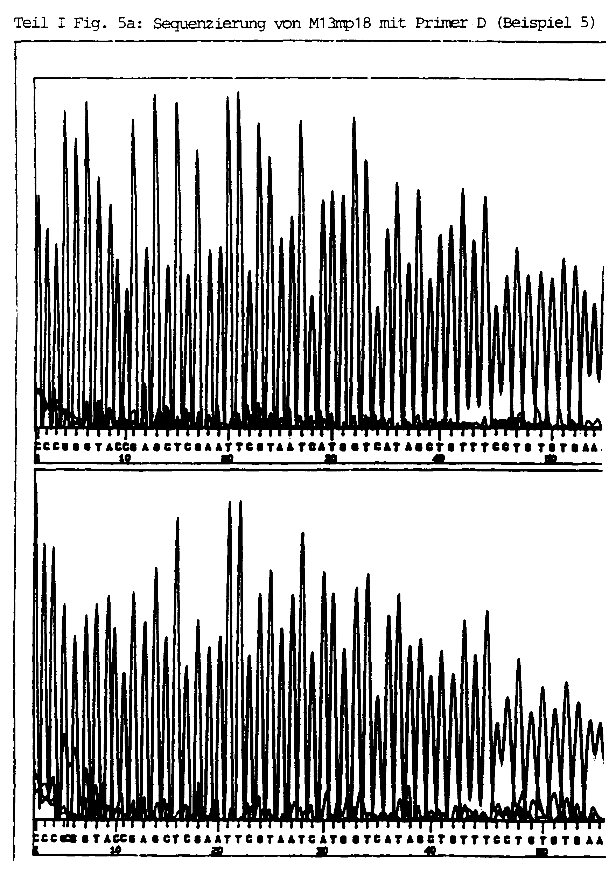

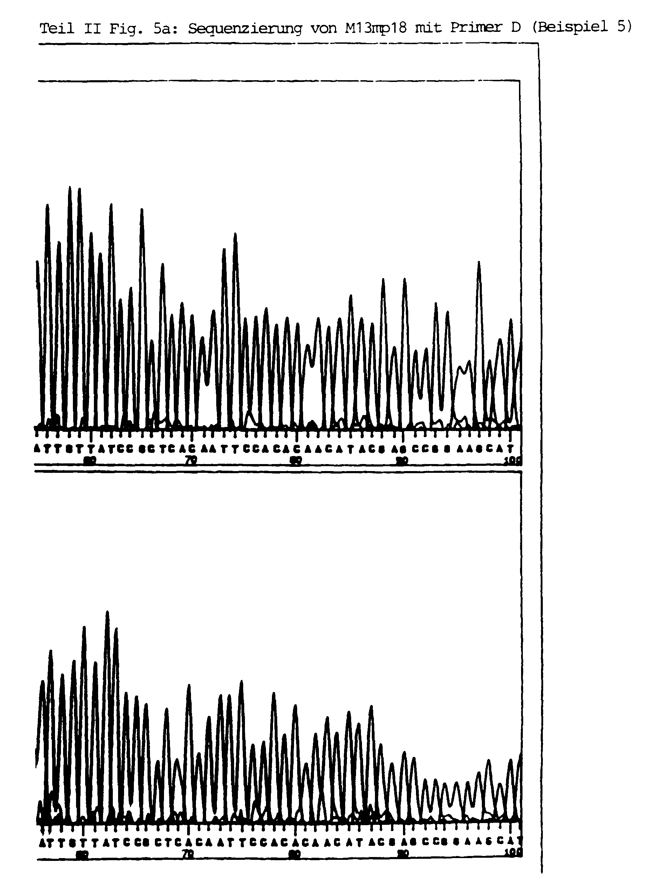

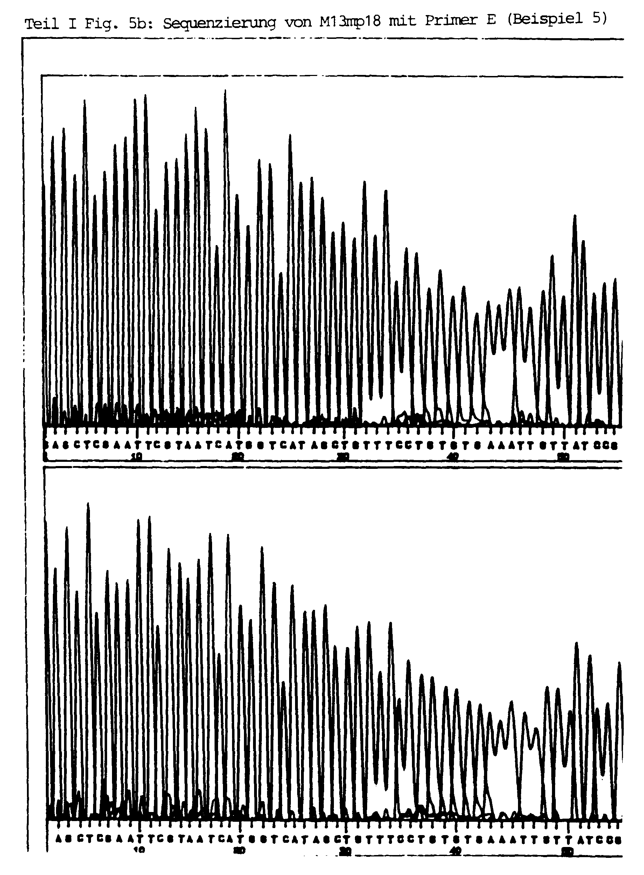

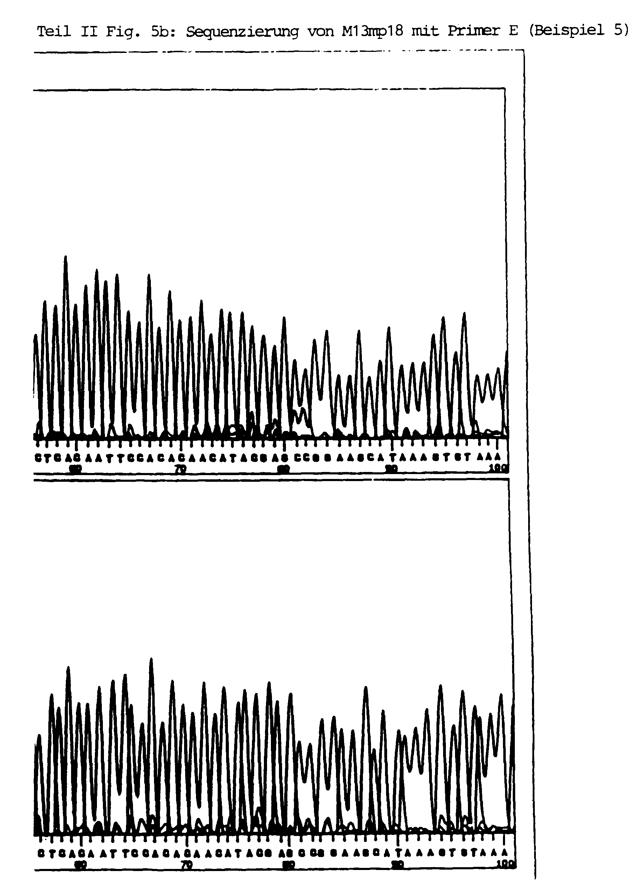

Die Durchführung der Sequenzierung erfolgte für beide Primer nach der Sanger'schen Didesoxymethode nach der Arbeitsvorschrift der Fa. Pharmacia (T7-Sequencing Kit). Die Sequenzierungsergebnisse sind in den Abbildungen 5a und b dargestellt.

### Sequenzprotokoll

SEQ ID NO 1
   - Sequenzlänge: 17 Basen
   - Art der Sequenz: Nukleotid
   - Strangform: Einzelstrang
   - Topologie: Linear
   - Merkmal: C an 3'-Ende fluoresceinmarkiert CAGGAAACAG CTATAGC 17
SEQ ID NO 2
   - Sequenzlänge: 17 Basen
   - Art der Sequenz: Nukleotid
   - Strangform: Einzelstrang
   - Topologie: Linear
   - Merkmal: C am 3'-Ende fluoresceinmarkiert CAGTATCGAC AAAGGAC 17
SEQ ID NO 3
   - Sequenzlänge: 23 Basen
   - Art der Sequenz: Nukleotid
   - Strangform: Einzelstrang
   - Topologie: Linear
   - Merkmal: A am 5'-Ende fluoresceinmarkiert AGTATCGACA AAGGACACAC TTT 23
SEQ ID NO 4
   - Sequenzlänge: 17 Basen
   - Art der Sequenz: Nukleotid
   - Strangform: Einzelstrang
   - Topologie: Linear
   - Merkmal: A am 3'-Ende mit Fluoresceinmarkiert TTGTAAAACG ACGGCCA 17
SEQ ID NO 5
   - Sequenzlänge: 17 Basen
   - Art der Sequenz: Nukleotid
   - Strangform: Einzelstrang
   - Topologie: Linear
   - Merkmal: C am 3'-Ende mit Fluoresceinmarkiert

AAAACGACGG CCAGTGC 17

## Patentansprüche

1. Verwendung von Oligonukleotiden als Primer in matrizenabhängigen, durch DNA-oder RNA-Polymerase katalysierten Kettenverlängerungen ausgehend von deren 3'-Ende, dadurch gekennzeichnet, daß diese Oligonukleotide am 3'-Ende eine definierte Anzahl und Art von mit einer nicht radioaktiven Markierungsgruppe versehenen Desoxyribo- oder Ribo-Nukleosiden, - Nukleotiden oder Derivaten oder Vorstufen davon aufweisen.

2. Verwendung gemäß Anspruch 1, dadurch gekennzeichnet, daß die Reaktion Teil eines enzymatischen Nukleinsäuresequenzierungsverfahrens ist.

3. Verwendung gemäß Anspruch 1, dadurch gekennzeichnet, daß die Reaktion Teil der Polymerase Chain Reaction ist.

4. Verwendung gemäß Anspruch 1, dadurch gekennzeichnet, daß die Markierungsgruppe oder die Markierungsgruppen an die Nukleobase der Desoxyribo- oder Ribo- Nukleosdie, -Nukleotide oder Derivate oder Vorstufen davon gebunden sind.

5. Verfahren zur Herstellung von markierten Nukleinsäuren durch Hybridisieren eines Primers an eine Matrizennukleinsäure und enzymatische Verlängerung dieses Primers ausgehend von dessen 3'-Ende, dadurch gekennzeichnet, daß dieser Primer am 3'-Ende ein mit einer nicht radioaktiven Markierungsgruppe versehenes Nukleosid enthält.

6. Verfahren gemäß Anspruch 5, dadurch gekennzeichnet, daß die Markierungsgruppe an die Base des Nukleosids gebunden ist.

## Claims

1. Use of oligonucleotides as a primer in template-dependent chain elongations catalysed by DNA or RNA polymerase starting at their 3' end, wherein these oligonucleotides have a defined number and type of deoxyribo- or ribonucleosides, -nucleotides or derivatives or precursors thereof at the 3' end which are provided with a non-radioactive labelling group.

2. Use as claimed in claim 1, wherein the reaction is part of an enzymatic nucleic acid sequencing method.

3. Use as claimed in claim 1, wherein the reaction is part of the polymerase chain reaction.

4. Use as claimed in claim 1, wherein the labelling group or the labelling groups are bound to the nucleobase of the deoxyribo- or ribonucleosides,-nucleotides or derivatives or precursors thereof.

5. Process for the production of labelled nucleic acids by hybridization of a primer to a template nucleic acid and enzymatic extension of this primer starting at its 3' end, wherein this primer contains a nucleoside provided with a non-radioactive labelling group at its 3' end.

6. Process as claimed in claim 5, wherein the labelling group is bound to the base of the nucleoside.

## Revendications

1. Utilisation d'oligonucléotides en tant qu'amorces dans des allongements de chaîne catalysées par l'ADN polymérase ou l'ARN polymérase, dépendantes de la matrice, partant de leur extrémité 3', caractérisée par le fait que ces oligonucléotides présentent, à leur extrémité 3', un nombre défini de désoxyribo- ou ribo-nucléosides, nucléotides ou leurs dérivés ou précurseurs, de type défini, munis d'un groupe marqueur non radioactif.

2. Utilisation selon la revendication 1, caractérisée par le fait que la réaction fait partie d'un procédé de séquençage enzymatique d'acide nucléique.

3. Utilisation selon la revendication 1, caractérisée par le fait que la réaction fait partie de la réaction d'amplification en chaîne par polymérase (Polymerase Chain Reaction).

4. Utilisation selon la revendication 1, caractérisée par le fait que le ou les groupes marqueurs sont liés à la base nucléotidique des désoxyribo- ou ribo-nucléosides, nucléotides ou de leurs dérivés ou précurseurs.

5. Procédé de préparation d'acides nucléiques marqués, par hybridation d'une amorce à un acide nucléique matrice et allongement enzymatique de cette amorce en partant de son extrémité 3', caractérisé par le fait que cette amorce comprend, à son extrémité 3', un nucléoside muni d'un groupe marqueur non radioactif.

6. Procédé selon la revendication 5, caractérisé par le fait que le groupe groupe marqueur est lié à la base du nucléoside.
